# EUROPEAN PATENT SPECIFICATION

(11) **EP 0 595 811 B1**
(45) Date of publication and mention of the grant of the patent: **20.01.1999**
(21) Application number: 92902586.4
(22) Date of filing: 13.11.1991
(51) Int. Cl.: A61K 7/48, A61K 7/16

(54) **COSMETICAL PRODUCT**
KOSMETISCHES PRODUKT
PRODUIT COSMETIQUE

(30) Priority: 13.11.1990 SE 9003614
(43) Date of publication of application: 11.05.1994
(73) Proprietor: PROCUR AKTIEBOLAG, 222 53 Lund (SE)
(72) Inventor: NILSSON, Kurt, S-226 53 Lund (SE)
(74) Representative: Wiklund, Erik
(86) International application number: SE9100768
(87) International publication number: WO9208443

(56) References cited:
- EP-A- 296 620
- EP-A- 379 753
- WO-A-88/04168
- WO-A-91/00867
- CH-A- 429 026
- DE-A- 1 568 320
- DE-A- 3 816 522
- DE-C- 3 528 168
- GB-A- 857 243

## Description

The present invention relates to a cosmetic product or product aimed for application on skin or teeth, which contains at least one carbohydrate (or an analog thereof) which constitutes a fragment, or an analog, of the carbohydrate part in a glycoconjugate.

It has been found that the oligosaccharide part of various glycoconjugates (especially glycolipids and glycoproteins) have a number of important functions in vivo (Biology of Carbohydrates, vol. 2, Ginsburg et al., Wiley, New York, 1984; The Glycoconjugates, vol. I-V, Academic Press, New York; S. Hakomori, Ann. Rev. Biochem., vol. 50, pp. 733-64, Ann. Rev. Biochem. vol. 58, 309-350; Feizi, Nature, pp. 314, 1985; S. Hakomori, Chemistry and Physics of Lipids, vol 42, pp. 209-33). Among other things it was found that
- the carbohydrate structures are important for the stability, activity, localisation, immunogenicity and degradation of glycoproteins;
- carbohydrates are antigenic determinants (for example blood group antigens);
- carbohydrates function as receptors when bound to cell surfaces for pathogens, proteins, hormons, toxins and during cell-cell interactions;
- carbohydrates are important for oncogenesis, since specific oligosaccharides have been found to be cancer-associated antigenic determinants;
- frequently, only a smaller sequence (di- or trisaccharide) of the carbohydrate part of the glycoconjugate is required for full biological activity (e.g. receptor activity).

Universities and Industry are at present working intensely on developing the use of biologically active oligosaccharides within a number of different fields, such as
- novel diagnostics and blood typing reagents
- highly specific materials for affinity chromatography
- cell-specific agglutination reagents
- targetting of drugs
- monoclonal antibodies, specific against e.g. cancer-associated structures
- therapy
- development of a new type of therapy, as an alternative to antibiotics, based on the inhibition of the attachment of bacteria and virus on cell surfaces with specific oligosaccharides
- stimulation of the growth of plants and protection against pathogens.

Besides the above mentioned areas, a considerable future market is envisaged for fine chemicals based on biologically active carbohydrates.

About ten different monosaccharides are included in the carbohydrate part of the glycoconjugates: D-glucose (Glc), D-galactose (Gal), N-acetyl-D-glucosamine (GlcNAc), N-acetyl-neuraminic acid (Neu5Ac), D-mannose (Man), L-fucose (Fuc), N-acetyl-D-galactosamin (GalNAc), xylose (Xyl) and arabinose (Ara) (the abbreviations in brackets are according to IUPAC-IUB's abridged terminology for monosaccharides, J.Biol.Chem., vol 257, pp. 3347-3354, 1982, in which publication one also can find the nomenclature used in this text to describe oligosaccharide sequences). The number of possible structures is almost infinitely great because both the anomeric configuration and the position of the O-glycosidic bond can be varied.

The present invention describes a new type of cosmetic product as defined in claim 1 and relates to the use of it for the manufacture of a medicament for the inhibition of bacterial attachment.

EP-A-0 296 620 describes N-acetylneuraminic-acid salts as repairing agents for cells and tissues.

EP-A-0 379 753 describes N-acetylglucosamine preparations for buccal use.

WO 91/00867 describes pharmaceutical compositions containing a 5'-diphosphohexose nucleoside, i.e. a monosaccharide.

DE-A-3 816 522 describes a caries protection composition, inter alia containing the monosaccharide galactose.

CH-A-429 026 describes a method for the preparation of a saccharide extract from Cactus opuntia vulgaris for cosmetic use.

GB-A-857 243 describes pharmaceutical compositions for topical use comprising one or more pentoses and one or more amino acids.

DE-A-3 528 168 describes a cosmetic preparation comprising mono-, di- and oligosaccharides obtained from starch and without biological activity.

WO 88/04168 describes a topical cream containing mono- and disaccharide compounds, some of which have anti-bacterial activity due to an osmotic effect when used in high concentrations.

DE-A-1 568 320 describes a cosmetic preparation containing saccharides maintaining the water retention in the skin, but without any biological activity.

The cosmetic product can be in many different forms, e.g. in the form of a plaster, a soap, a cleansing cream, a tooth paste, in liquid form (e.g. cleansing water) or as a lotion or a cream ( e.g. night or day cream).

The carbohydrate or carbohydrate derivative function e.g. as an inhibitor for bacterial attachment to the skin or the teeth, as a moisturizer, or as a nutritional supplement for the skin in the production of protecting, moisturizing carbohydrates.

Examples of nucleotide sugars are UDP-Gal, CMP-NeuAc, UDP-GalNAc, GDP-Fuc, etc. These can be produced preferentially with biotechnological methods involving enzymes, e.g. CMP-sialate synthase and other enzymes for synthesis of nucleotide sugars.

Examples of carbohydrate structures of glycoconjugates are found in the references on p. 1 and in the tables below. Of special interest are the minutest fragments - often di-, tri- or tetrasaccharides or analogs - of these structures, which are sufficient to transfer biological activity.

Examples of interesting structures are blood group determinants, cancer-associated oligosaccharide structures and structures with bacterial receptor activity (see references on page 1 above).

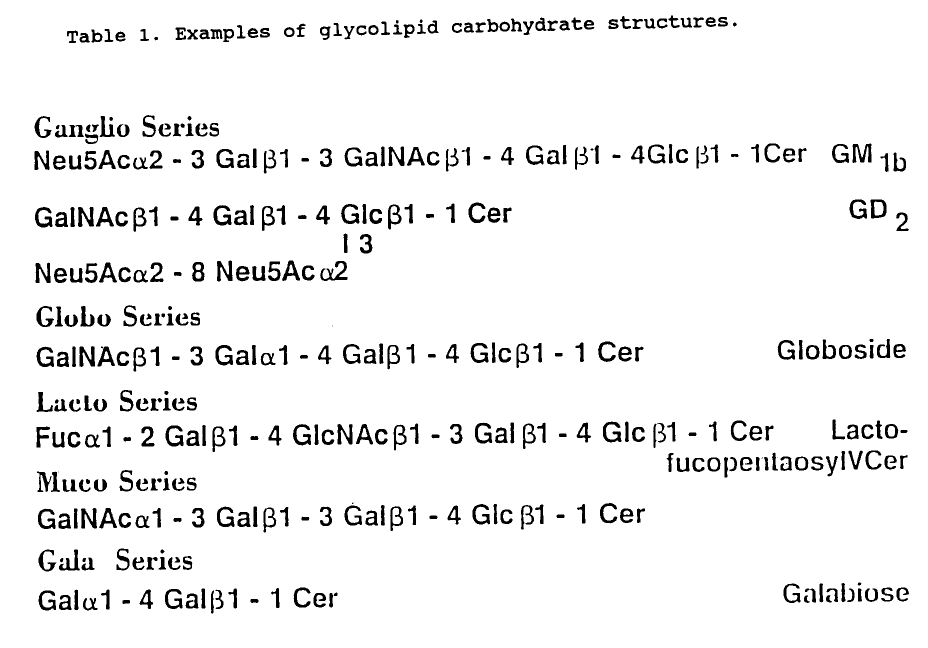

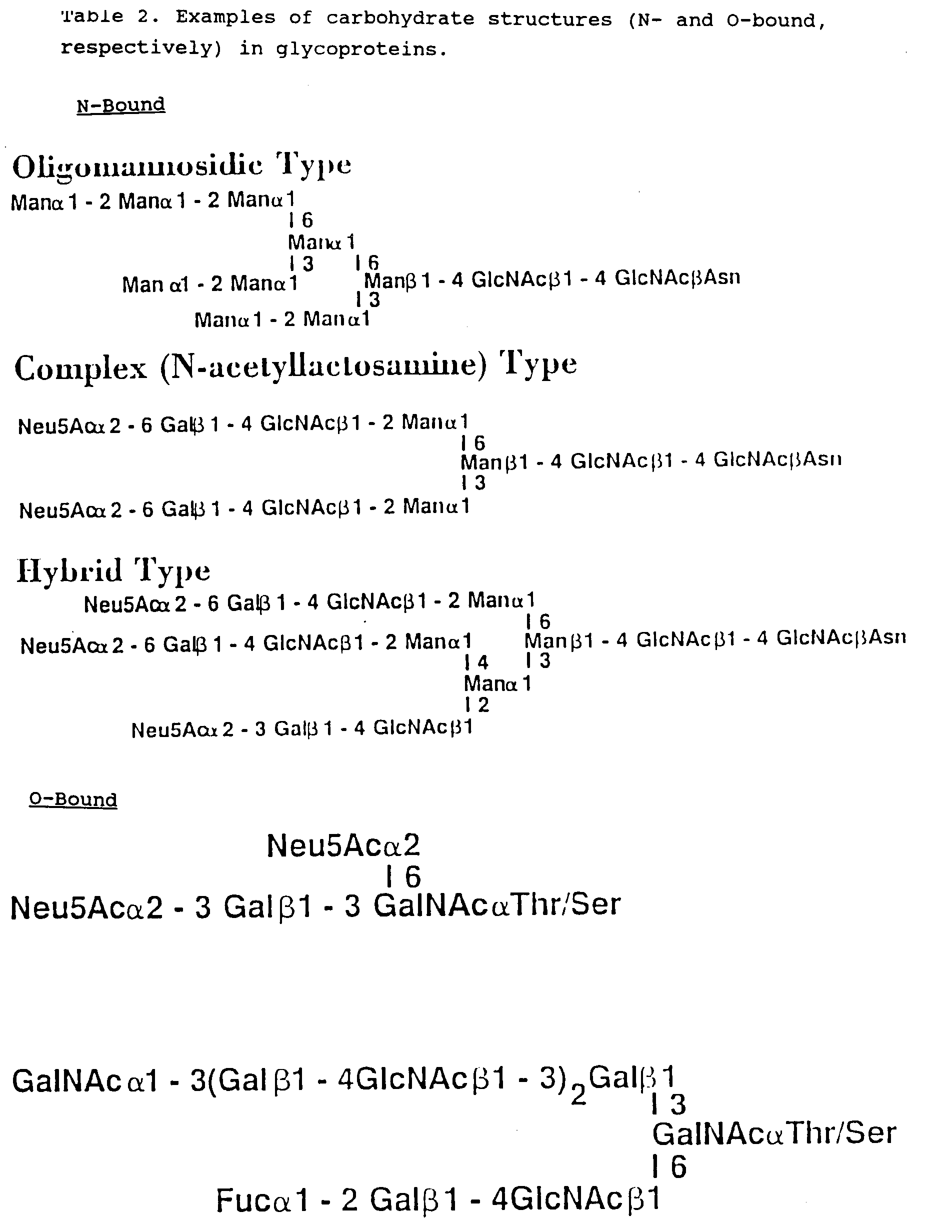

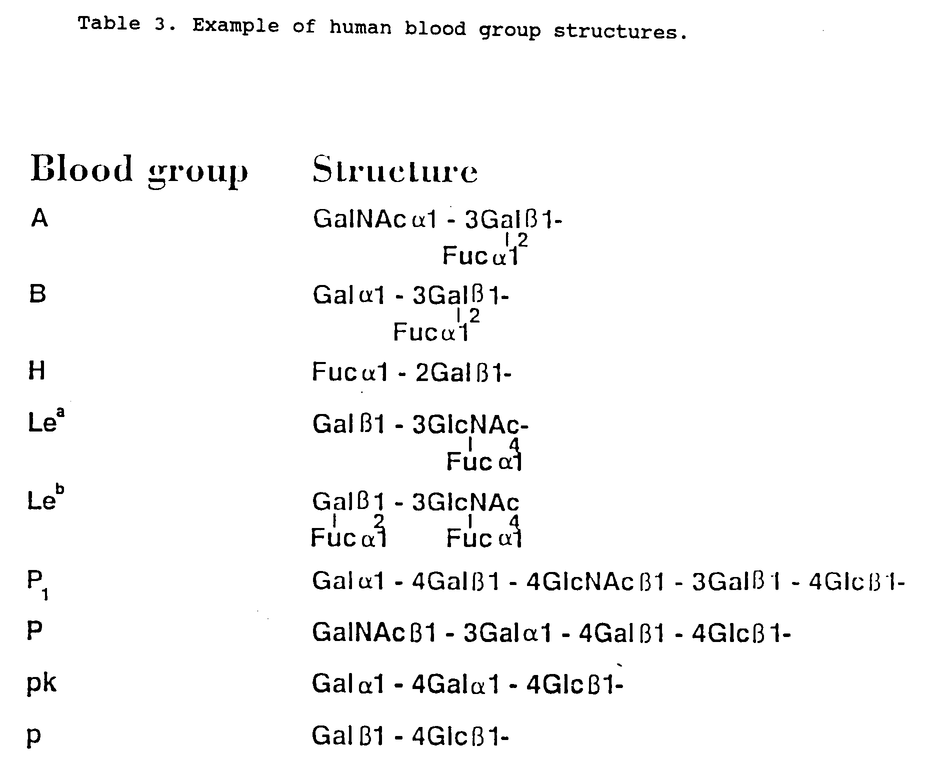

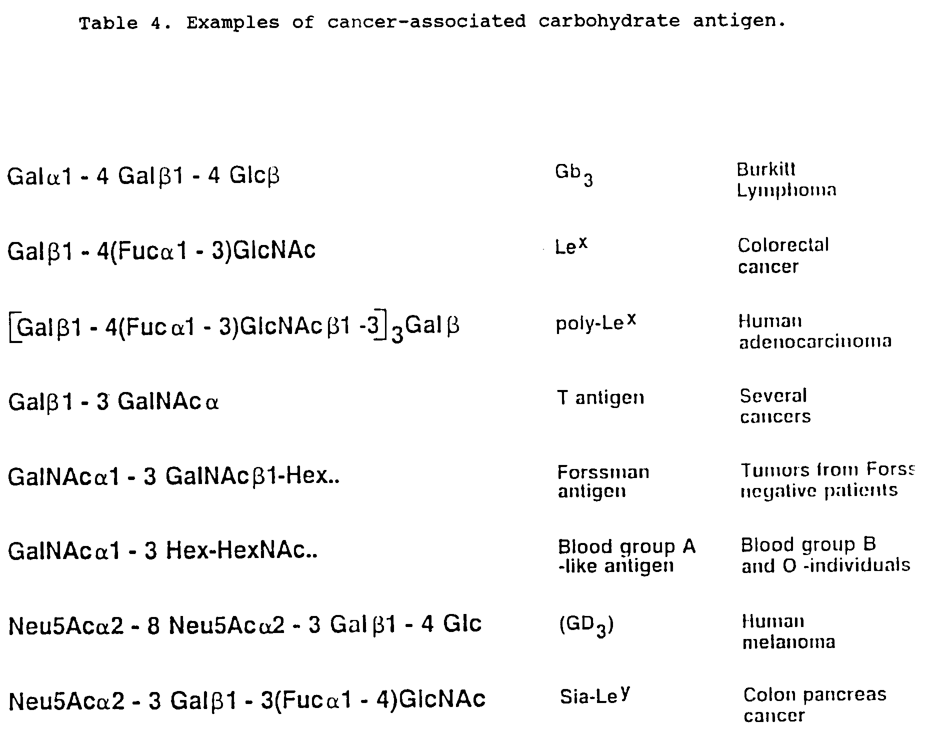

The carbohydrate is produced according to the invention preferentially by biotechnological methods employing glycosidase and/or glycosyltransferase.

Both hydrolases (glycosidases, EC 3.2) and glycosyl-transferases (EC 2.4) can be used for synthesis (glycosidases: see Nisizawa et al, in "The Carbohydrates, Chemistry and Biochemistry", 2nd ed., vol IIA, pp. 242-290, Academic Press, New York, 1970). With glycosidases reversed hydrolysis (equilibrium reaction) or transglycosylation (kinetic reaction) are often used to obtain synthesis (see e.g. K.G.I. Nilsson, Carbohydr. Res., vol. 167, pp. 95-103, 1987, Trends in Biotechnology., vol. 6, pp. 256-264, 1988).

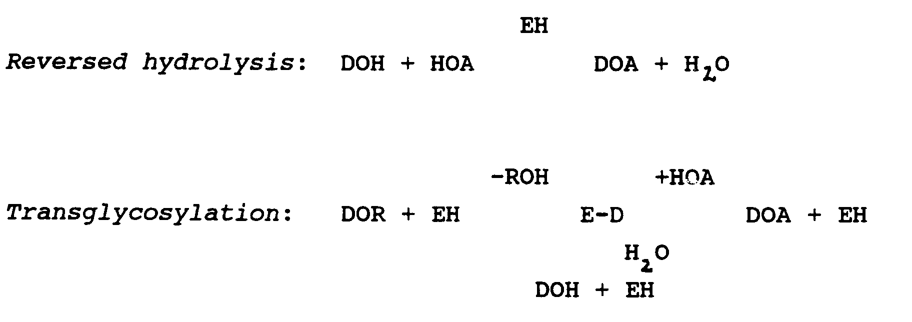

(DOH is donor saccharide, DOR is donor glycoside with - or β-glycosidically bound aglycon (=R), HOA is acceptor saccharide and EH is enzyme).

With transferases, a nucleotide sugar (UDP-Gal, CMP-Sia, UDP-GalNAc, GDP-Fuc, etc), which is relatively expensive, is used as donor. Furthermore, glycosidases are abundant and can often be used directly without purification. A disadvantage is however that relatively low yields and the wrong product isomers often are obtained.

## Claims

1. Cosmetic product or a product for application on the skin or on the teeth containing one or more ingredient(s) conventionally used in such products, **characterised** in that it also contains a di-, tri-or tetrasaccharide fragment of the carbohydrate part in a glycoprotein or a glycolipid, and optionally at least one nucleotide sugar.

2. Product according to claim 1, **characterised** in that the di, tri- and tetrasaccharide have been produced by a biotechnological method employing a glycosidase and/or a glycosyl transferase.

3. Product according to any of the previous claims, **characterised** in that the carbohydrate part of the glycoprotein is

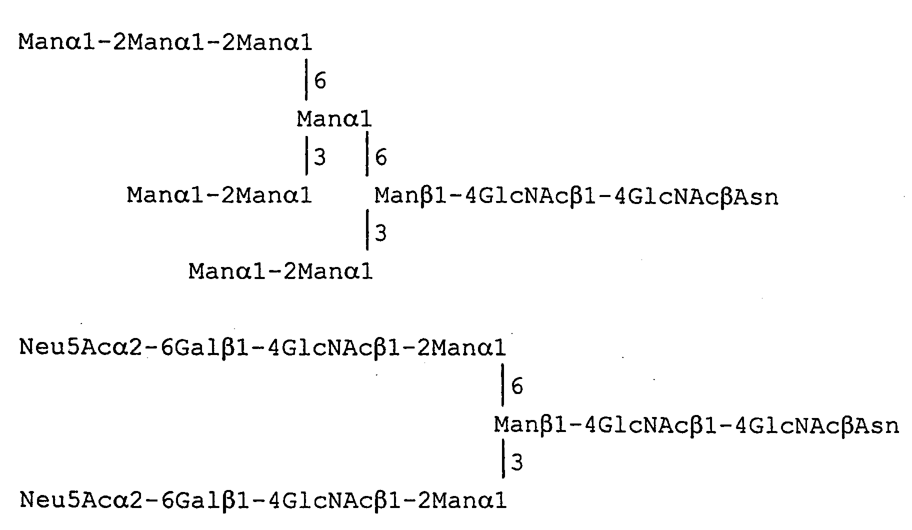

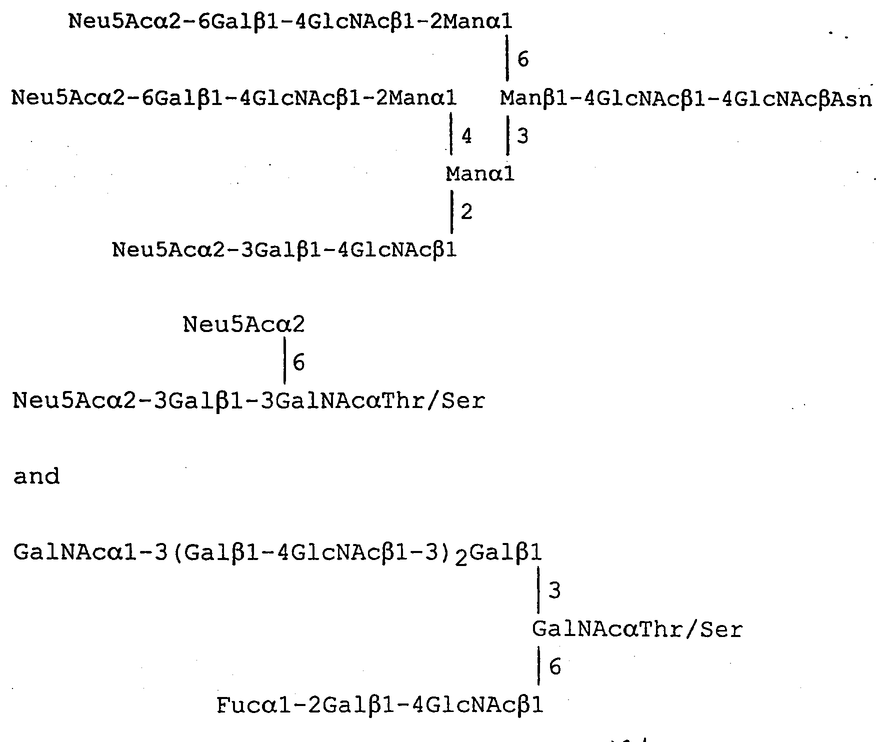

4. Product according to claims 1 and 2, **characterised** in that the carbohydrate part of the glycolipid is
Neu5Acα2-3Galβ1-3GalNAcβ1-4Galβ1-4Glcβ1-1Cer 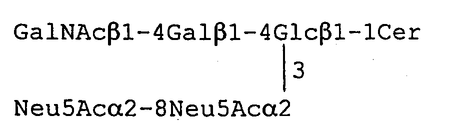 GalNAcβ1-3Galα1-4Galβ1-4Glcβ1-1Cer
Fucα1-2Galβ1-4GlcNAcβ1-3Galβ1-4Glcβ1-1Cer
GalNAcα1-3Galβ1-3Galβ1-4Glcβ1-1Cer
and
Galα1-4Galβ1-1Cer.

5. Product according to any of the previous claims, **characterised** in that the nucleotide sugar is UDP-Gal, CMP-Sia, CMP-NeuAc, UDP-GalNaC and/or GDP-Fuc.

6. Product according to claim 5, **characterised** in that the nucleotide sugar has been produced by use of a nucleotide sugar synthesis enzyme.

7. Product according to claim 6, **characterised** in that the nucleotide sugar synthesis enzyme is CMP-sialate synthase.

8. Product according to claim 1, **characterised** in that the di-, tri- or tetrasaccharide is or constitutes a fragment of the human blood group structures 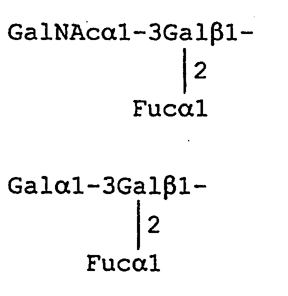 Fucα1-2Galβ1- 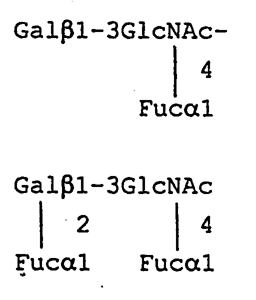 Galα1-4Galβ1-4GlcNAcβ1-3Galβ1-4Glcβ1-
GalNAcβ1-3Galα1-4Galβ1-4Glcβ1-, and
Galα1-4Galα1-4Glcβ1-
and/or a cancer-associated carbohydrate antigen structure
Galα1-4Galβ1-4Glcβ,
Galβ1-4(Fucα1-3)GlcNac,
(Galβ1-4(Fucα1-3)GlcNAcβ1-3)₃Galβ,
Galβ1-3GalNAcα,
GalNAcα1-3GalNAcβ1-Hex..,
GalNAcα1-3Hex-HexNAc..,
Neu5Acα2-8Neu5Acα2-3Galβ1-4Glc, and
Neu5Acα2-3Galβ1-3(Fucα1-4)GlcNAc.

9. Product according to any of the previous claims, **characterised** in that it is administrated in the form of a plaster, a soap, a cleansing cream, a tooth paste, in liquid form, as cleansing water, or as a lotion or a cream, as a night or day cream.

10. Use of a product according to claim 1 for the manufacture of a medicament for the inhibition of bacterial attachment.

## Patentansprüche

1. Kosmetisches Erzeugnis oder Erzeugnis zur Anwendung auf die Haut oder auf die Zähne, enthaltend einen oder mehrere herkömmlicherweise in solchen Erzeugnissen verwendete(n) Bestandteil(e), **dadurch gekennzeichnet,** daß es auch ein Di-, Tri- oder Tetrasaccharidfragment des Kohlenhydratteils in einem Glycoprotein oder einem Glycolipid und gegebenenfalls mindestens einen Nukleotidzucker enthält.

2. Erzeugnis nach Anspruch 1, **dadurch gekennzeichnet,** daß das Di-, Tri- und Tetrasaccharid durch ein biotechnologisches Verfahren unter Verwendung einer Glycosidase und/oder einer Glycosyltransferase hergestellt worden ist.

3. Erzeugnis nach einem der vorangehenden Ansprüche, **dadurch gekennzeichnet**, daß der Kohlenhydratteil des Glycoproteins

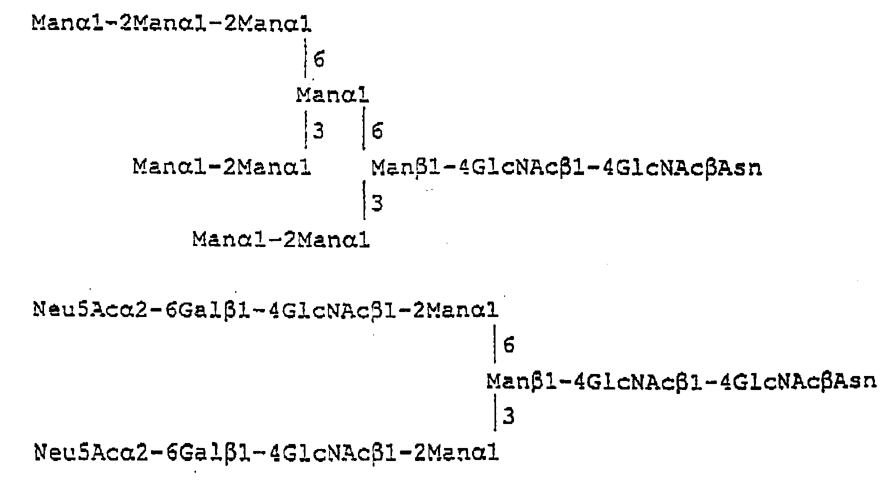

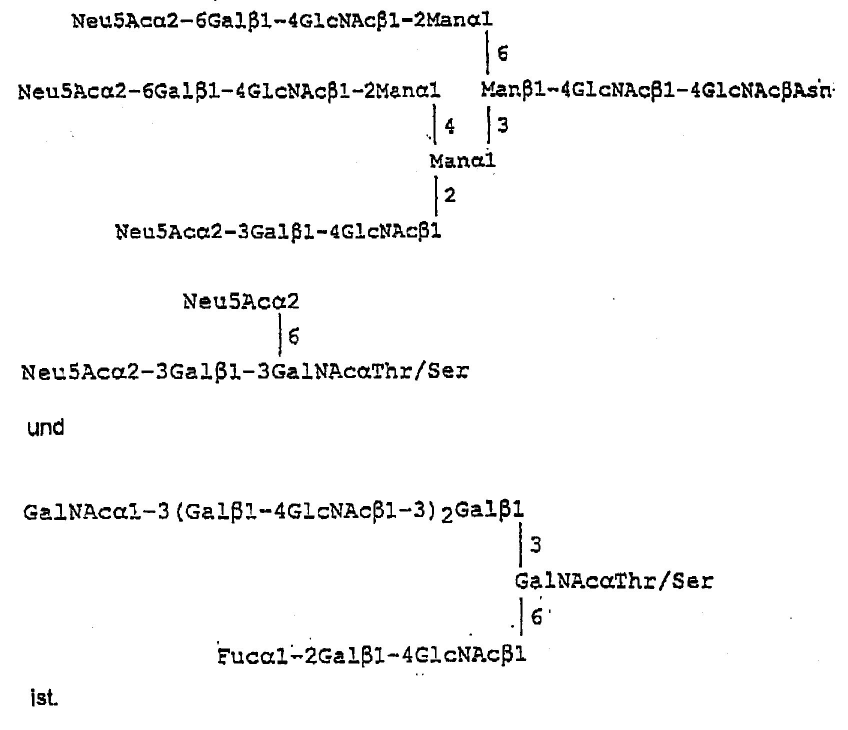

4. Erzeugnis nach den Ansprüchen 1 und 2, **dadurch gekennzeichnet,** daß der Kohlenhydratteil des Glycolipids
Neu5Acα2-3Galβ1-3GalNAcβ1-4Galβ1-4Glcβ1-1Cer 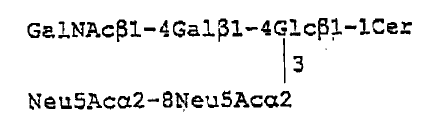 GalNAcβ1-3Galα1-4Galβ1-4Glcβ1-1Cer
Fucα1-2Galβ1-4GlcNAcβ1-3Galβ1-4Glcβ1-1Cer
GalNAcα1-3Galβ1-3Galβ1-4Glcβ1-1Cer
und
Galα1-4Galβ1-1Cer
ist.

5. Erzeugnis nach einem der vorangehenden Ansprüche**, dadurch gekennzeichnet**, daß der Nukleotidzucker UDP-Gal, CMP-Sia, CMP-NeuAc, UDP-GalNaC und/oder GDP-Fuc ist

6. Erzeugnis nach Anspruch 5, **dadurch gekennzeichnet,** daß der Nukleotidzucker durch die Verwendung eines Nukleotidzuckersyntheseenzyms hergestellt worden ist.

7. Erzeugnis nach Anspruch 6, **dadurch gekennzeichnet,** daß das Nukleotidzuckersyntheseenzym CMP-Sialat-Synthase ist.

8. Erzeugnis nach Anspruch 1, **dadurch gekennzeichnet**, daß das Di-, Tri- oder Tetrasaccharid ein Fragment der menschlichen Blutgruppenstrukturen 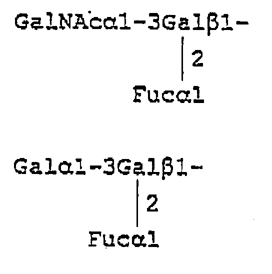 Fucα1-2Galβ1- 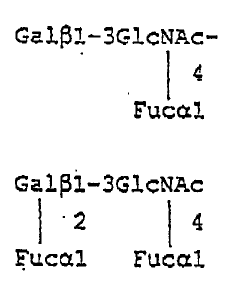 Galα1-4Galβ1-4GlcNAcβ1-3Galβ1-4Glcβ1-
GalNAcβ1-3Galα1-4Galβ1-4Glcß1-
und
Galα1-4Galα1-4Glcβ1-
und/oder eine krebs-assoziierte Kohlenhydrat-Antigenstruktur
Galα1-4Galβl-4Glcβ,
Galβ1-4 (Fucα1-3) GlcNac,
(Galβ1-4(Fucα1-3)GlcNAcβ1-3)₃Galβ,
Galβ1-3GalNAcα,
GalNAcα1-3GalNAcβ1-Hex..,
GalNAcα1-3Hex-HexNAc..,
Neu5Acα2-8Neu5Acα2-Galβ1-4Glc,
und
Neu5Acα2-3Galβ1-3(Fucα1-4)GlcNAc
ist oder bildet.

9. Erzeugnis nach einem der vorangehenden Ansprüche**, dadurch gekennzeichnet,** daß es In Form eines Pflasters, einer Seife, einer Reinigungscreme, einer Zahnpasta, in flüssiger Form, als wäßrige Reinigungslösung oder als Lotion oder Creme, als Nacht- oder Tagescreme verabreicht wird.

10. Verwendung eines Erzeugnisses nach Anspruch 1 zur Herstellung eines Arzneimittels zur Verhinderung des Anhaftens von Bakterien.

## Revendications

1. Produit cosmétique ou produit pour l'application sur la peau ou sur las dents contenant un ou plusieurs ingrédients utilisés traditionnellement dans de tels produits, caractérisé en ce qu'il contient aussi un fragment di-, tri- ou tétrasaccharide d'une partie de glucide dans une glycoprotéine ou un glycolipide, et éventuellement au moins un sucre nucléoside,

2. Produit selon la revendication 1, caractérisé en ce que le di-, tri- et tétrasaccharide ont été préparés par un procédé biotechnologique utilisant une glycosidase et/ou une glycosyltransférase.

3. Produit selon l'une quelconque des revendications précédentes, caractérisé en ce que la partie glucide dans la glycoprotéine est

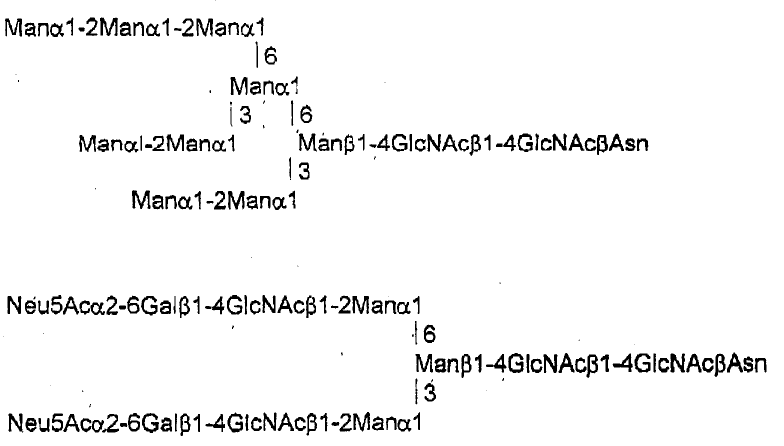

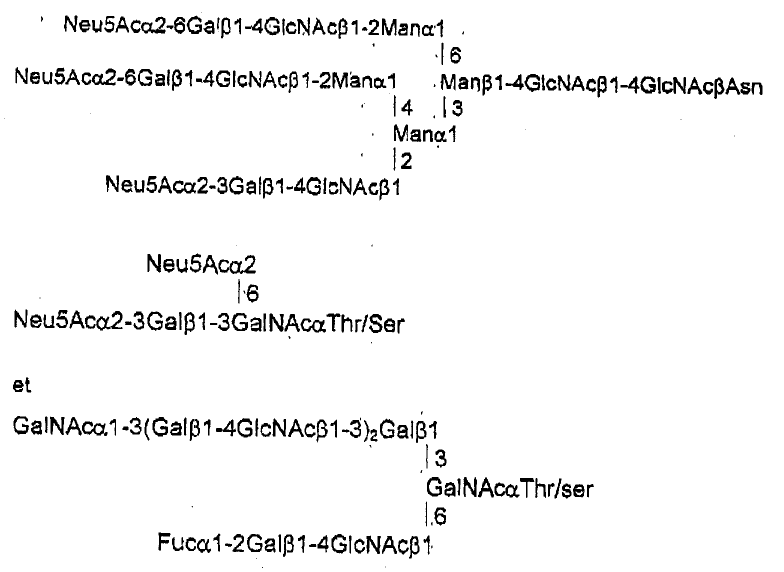

4. Produit selon la revendication 1 et 2, caractérisé en ce que le la partie glucide du glycolipide est
Neu5Acα2-3-Galβ1-3GalNacβ1-4Galβ1-4Glcβ1-1Cer 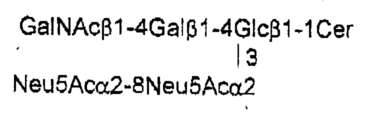 GalNAcβ1-3Galα1-4Gaβ1-4Glcβ1-1Cer
Fucα1-2Galβ1-4GlcNAcβ1-3Galβ1-4Glcβ1-1Cer
GalNAcα1-3Galβ1-3Galβ1-4Glcβ1-1Cer
et
Galα1-4Galβ1-1Cer

5. Produit selon l'une quelconque des revendications précédentes, caractérisé en ce que le sucre nucléotide est UDP-Gal, CMP-Sia, CMP-NeuAc, UDP-GalNaC et/ou GDP-Fuc.

6. Produit selon la revendication 5, caractérisé en ce que le sucre nucléotide a été préparé en utilisant une enzyme de synthèse de sucre nucléotide.

7. Produit selon la revendication 6, caractérisé en ce que l'enzyme de synthèse de sucre nucléotide est la synthase CMP-sialate.

8. Produit selon la revendication 1, caractérisé en ce que le di-, tri- ou le tétrasaccharide est, ou constitue, un fragment de structure du groupe sanguin humain 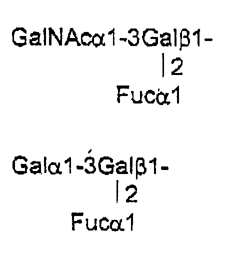 Fucα1-2Galβ1- 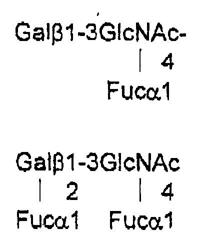 Galα1-4Galβ1-4GlcNAcβ1-3Galβ1-Glcβ1-
GalNAcβ1-3Galα1-4Galβ1-4Glcβ1- et
Galα1-4Galα1-4Glcβ1
et/ou une structure d'antigène glucide associée au cancer
Galα1-4Galβ1-4Glcβ,
Galβ1-4(Fucα1-3)GlcNac,
(Galβ1-4(Fucα1-3)GlcNAcβ1-3)₃Galβ,
Galβ1-3GalNAcα,
GalNAcα1-3GalNAcβ1-Hex..,
GalNAcα1-3Hex-HexNAc..,
Neu5Acα2-8Neu5Acα2-3Galβ1-4Glc, et
Neu5Acα2-3Galβ1-3(Fucα1-4)GlcNAc.

9. Produit selon l'une quelconque des revendications précédentes, caractérisé en ce qu'il est administré sous forme d'un emplâtre, d'un savon, d'une crème nettoyante, d'une pâte dentifrice, sous forme liquide, comme eau nettoyante, ou comme une lotion ou une crème, comme une crème de nuit ou de jour.

10. Utilisation d'un produit selon la revendication 1 pour la fabrication d'un médicament pour l'inhibition de la fixation bactérienne.
